# EUROPEAN PATENT SPECIFICATION

(11) **EP 1 200 017 B1**
(45) Date of publication and mention of the grant of the patent: **05.01.2005**
(21) Application number: 00954575.7
(22) Date of filing: 28.07.2000
(51) Int. Cl.: A61F 2/06

(54) **STENT DEPLOYMENT CATHETER WITH SHEATH SEPARATING DEVICE**
STENTANBRINGUNGSKATHETER MIT VORRICHTUNG ZUM TRENNEN EINER HÜLLE
CATHETER DE DEPLOIEMENT D'EXTENSEUR AVEC DISPOSITIF SEPARATEUR DE GAINE

(30) Priority: 02.08.1999 DE 19936207
(43) Date of publication of application: 02.05.2002
(73) Proprietor: Angiomed GmbH & Co. Medizintechnik KG, 76227 Karlsruhe (DE)
(72) Inventor: KOCH, Guido, D-76131 Karlsruhe (DE)
(74) Representative: HOFFMANN - EITLE
(86) International application number: PCT/EP2000/007320
(87) International publication number: WO 2001/008599

(56) References cited:
- EP-A- 0 732 087
- WO-A-98/20812
- FR-A- 2 688 688

## Description

### Technical Field

The present invention relates to a catheter for introducing and placing an endoprosthesis in a human or animal body, an application instrument having such a catheter as well as the use of a catheter for placing an oesophagus stent.

### State of the art

A stent placement system is known from French application 2 688 688, in which an endoprosthesis is received in the distal end region of a catheter. In the known system, three cutting wires spaced uniformly around the periphery of the catheter run in a loop from the proximal end region of a catheter to its distal end region and back again to the proximal end region. In the proximal end region of the catheter, both wires enter the shaft and then run within the shaft to emerge from the shaft in front of the expanding distal end region. One of the two guide wires re-enters in the expanded end region, the other runs past it externally. Both wires are guided parallel to one another past body tissue already changed pathologically in any case over a certain long region.

A balloon catheter having an intraluminal protective sheath is known from European application 0 732 087. A rigid collar, to which the protective sheath is attached distally and surrounds the tip of the catheter, sits in the distal end region of the known catheter. A cutting thread, which extends distally, at the tip of the protective tube emerges from the latter, is deflected and then runs coaxially to the catheter outside the protective sheath and collar and emerges from the body proximally with the latter, is also attached to the rigid collar within the sheath.

WO 98/20812 discloses a stent deployment system in which a sleeve encloses a stent carried on an inner catheter. The stent can be self-expandable or balloon-expandable. Upon deployment of the stent, the sleeve can be split by looping around a suture on the portion of the sleeve corresponding to the receiving region of the stent and pulling both ends of the suture proximally to open the sleeve as the suture travels proximally down the catheter sleeve.

All of these known constructions have in common the fact that a cutting element has to be guided in a gliding movement past body tissue to advantageously use the devices and to place a stent at the required point. However, it is inherent in the properties of the cutting elements that they exert a cutting effect during such a gliding movement and possibly damage body tissue.

### Object of the invention

The technical problem consequently underlying the present invention is to render possible devices, by means of which placing of an endoprosthesis is facilitated using low manual force, without, when placing the endoprosthesis, exposing tissue surrounding it later to the danger of possible damage.

### Summary of the invention

This technical problem is solved according to the present invention by a catheter having the features of claim 1 and by an application instrument having the features of claim 15. Advantageous further embodiments of the invention are given in the dependent claims.

Accordingly, the catheter of the invention serves for introducing an endoprosthesis into a human or animal body. The endoprosthesis can be received in one region of the catheter. The endoprosthesis is conventionally situated in this receiving region of the catheter in its compressed state. According to the invention the catheter is surrounded externally by a sheath. Within the scope of the present description of this invention, "externally" is understood to mean the side facing the surrounding tissue when the catheter is introduced. According to the invention the catheter likewise comprises a device for separating the sheath. This separating device has according to the invention a pull element and a separating element. According to the present invention for the first time for the means for separating the sheath of a catheter, there is differentiation between functionally different parts of this device, which are designed to be correspondingly different - as described below.

According to a first aspect of the invention, the separating device comprises an inner pull element, an outer pull element and the said separating element. A tensile force can be transferred to the separating element via the two pull elements. The inner pull element runs within the sheath in the region of the catheter which can be introduced. This ensures that the inner pull element cannot mechanically act directly on body tissue. At the same time clear and secure guiding of the inner pull element is guaranteed. By appropriate selection of the materials, on which only the mechanical requirement of tensile strength is placed, the friction coefficient can be influenced as required, so that for example the manual force necessary when placing an endoprosthesis is minimised. The outer pull element runs outside the sheath only in the receiving region for the endoprosthesis. Correspondingly, here too endangering body tissue is largely prevented, since the region of the pull element running outside the sheath is minimised. Parallel guiding of two cutting wires past body tissue known from the state of the art, which may squeeze body tissue between them under tensile stress, is advantageously prevented. According to the first aspect of the invention, when actuating the separating device, the inner pull element, the outer pull element and the separating element can also be moved together relative to the sheath to separate the sheath. This embodiment offers the advantage that the separating device may only be designed with the separating element of the invention over a small region of its length. Conventionally, this length corresponds approximately to the wall thickness of the sheath. By moving the inner and outer pull elements and the separating element together, these elements do not change their position relative to one another, and when separating the sheath essentially the same region of the separating device is engaged with the sheath. A movement of cutting devices or separating devices directly past body tissue is prevented.

Also, possibly damaging engagement between cutting wires known from the state of the art with the surrounding body tissue is minimized. According to the present invention, the pull element provided runs outside the sheath in the receiving region for the endoprosthesis and is designed to have low shearing effect in its region running outside the sheath, such that mechanical damage to the tissue surrounding the catheter is prevented. The functional separation and the corresponding design between pull element and separating element, which is realized for the first time according to the invention, becomes clear here again. In order to achieve separation of the sheath, the separating element deploys a separating effect, which is conventionally achieved by shear forces, cutting forces or the like. According to the invention, the pull element is designed such that such an action is essentially not deployed in its region running outside the sheath. It is designed to have a low shearing effect.

Due to the present invention devices are achieved which render possible the design of a catheter, in which a conceivably smooth actuation is combined with good separating capacity, since on the one hand the friction coefficient may be designed to be favourable over the greatest length range of the separating device, on the other hand there is no relative movement between inner and outer pull element, so that a design of these pull elements is possible such that they do not deploy a separating effect.

Devices, in which several cutting wires are used to separate the sheath, are often shown in the state of the art. Such multiplication of the cutting wires is proved to be advantageous for simple and reliable release of the endoprosthesis from the sheath. Within the scope of the present invention, investigations have been carried out with the surprising result that the manageability and the reliability during placement of a stent as regards positional accuracy and the release of the endoprosthesis from the sheath was best when only one single separating device is provided in the catheter of the invention. During the inventive separation it is possible for the stent to leave the sheath laterally, and to be anchored in the tissue over an uninterrupted and considerable part of its periphery. The part which is not anchored corresponds, as in the state of the art, to the periphery of the sheath, but influences according to the invention only a small portion of the stent periphery. However, the traditional multiple division of the sheath on the other hand ensures that the stent remains lying concentrically to the divided sheath after triggering and also appears to ensure that the proximal end region of the stent is constricted at several points - likewise concentrically as regards total effect - and may in comparison to the present invention be anchored only slightly.

Particularly simple production of the catheter of the invention results when the separating element is a cutting wire, which is provided according to a preferred further development of the invention.

The common movement of the parts provided according to the invention of the separating device of the invention is achieved when the separating device runs in a loop from a proximal end region to a distal end region of the catheter, around a deflecting point and back to the proximal end region of the catheter. Assembly may be realised simply by threading the separating device in the catheter according to this path, wherein exact alignment of the separating element relative to the sheath is guaranteed. The deflecting point is advantageously directly distally next to a distal end of the sheath, that is it is situated directly against the distal head edge of the sheath, so that when actuating the separating device no empty path has to be bridged and more precise operation is guaranteed.

However, the separating element particularly preferably is fixed tangentially just proximally of the distal end of the sheath in positive sheathing. Firstly, this ensures that even for rotary movement of the system, its position on the sheath periphery cannot change, secondly, the separating element can never be hooked in the stent during the separating process, since at the start of the cut it already lies just below the stent end.

In order to be able to separate the sheath in a simple pull movement in the proximal direction, according to a further embodiment of the invention provision is made in that the separating element is arranged at the deflecting point.

The low shearing action effect of the region of the pull element, which runs outside the sheath, can be realised in several ways. A particularly simple production, which reliably achieves the required protection of the surrounding tissue, was achieved according to the invention in that this region of the pull element is covered, preferably by a tube. When separating the sheath, this cover conventionally runs into the sheath with the pull element. When the receiving region is completely separated, consequently no components are situated outside the sheath, which could possibly injure surrounding tissue when the catheter is withdrawn. The required protective function is also guaranteed for unexpected movement paths of the pull elements by shape-precise covering by means of the tube provided according to the invention.

For some applications of the present invention, it is conceivable that a considerable length of the catheter is introduced into the human or animal body. This introduced length often runs around different radii of curvature. In order to guarantee best possible positional accuracy of the separating device relative to sheath, a further development of the invention makes provision in that the catheter comprises an inner guide element and the separating device runs proximally of the receiving region for the endoprosthesis next to the inner guide element. Guiding the catheter around a curve is thus associated with as low as possible a relative length change between sheath and separating device.

A further design of the present invention makes provision in that a control device displaying the end of separation positively communicates to the operating personnel that the sheath is reliably and completely separated.

Provision is also made according to the invention to provide an application instrument having a catheter of the invention and also an endoprosthesis. Such an application instrument typically comprises on the proximal side a device for manipulating the catheter, the catheter itself and possibly further devices and connecting pieces for supplying and removing fluids or the like.

The application instrument can be used particularly advantageously for placement of an oesophagus stent. This use is advantageous, since oesophagus stents have comparatively large dimensions and have to find space in a sheath that is narrow because of conventionally provided covers or coatings. In this inventive use, the high forces for placement resulting from these conditions for traditional catheters can be reduced such that only about 1/10 of the traditional triggering force is produced during placement.

### Brief description of the drawings

An exemplary embodiment is described and realised in more detail below for further illustration and for better understanding of the invention using the attached drawing.

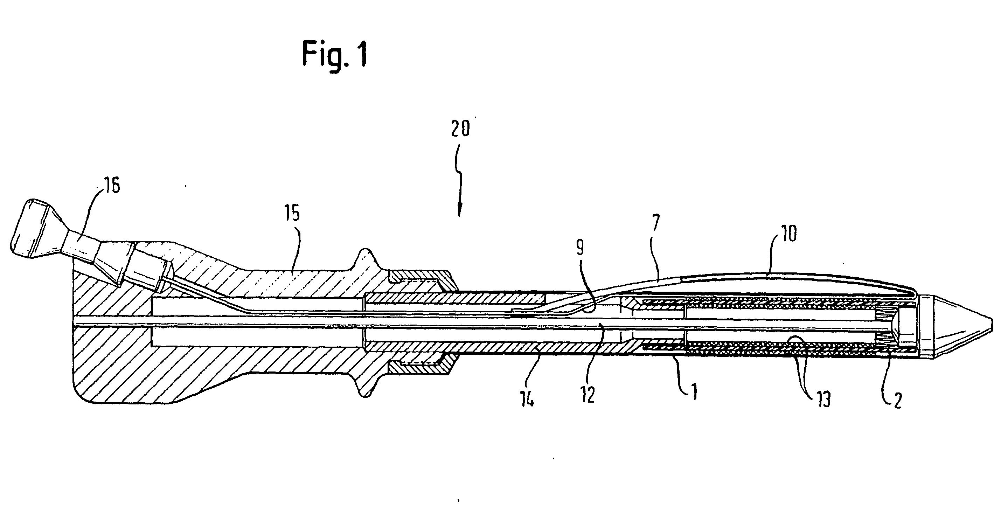

Figure 1 shows a sectioned side view of a first exemplary embodiment of the invention.

### Description of the embodiments of the invention

Figure 1 shows a first exemplary embodiment of the invention in half section in an assembly view.

This figure shows from left to right a handle piece 15 having a trigger mechanism 16 and a catheter not marked in more detail, which together form an application instrument. The catheter essentially comprises an inner guide element 12, conventionally an inner catheter or guide wire, which is received in a lumen of the catheter, and extends coaxially to the latter. Externally the catheter is surrounded by a sheath 1 which is arranged next to the body tissue when the catheter is introduced. An integrated filler 14, which is tapered in the region of the endoprosthesis designated by 2, to form a proximally clearly defined receiving space for the latter, is situated between the sheath 1 and the inner guide element 12 in the exemplary embodiment of Figure 1. The endoprosthesis in the exemplary embodiments shown is surrounded in regions by a stent coating 13, as is conventional for oesophagus stents.

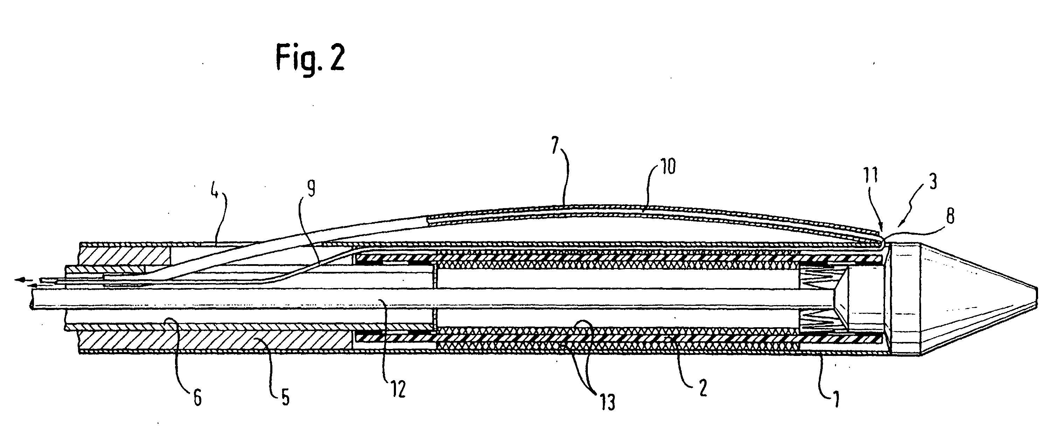

Figure 2 likewise in half section shows details of a further exemplary embodiment which comprises the following components from the inside to the outside:
as in the first embodiment, an inner guide element 12; proximally a lumen not marked in more detail in which this inner guide element 12 runs; differing from the first embodiment an inner filler tube 6 and proximally an outer filler tube 5, which is attached axially to be fixed in position on the inner filler tube 6 and analogously to the tapering of the first embodiment, forms a proximal contact edge for the endoprosthesis 2 arranged distally in front of it; as in the first embodiment outside the endoprosthesis 2 an inner pull element 9, a sheath 1 and finally an outer pull element 10 with a protective tube 7.

The endoprosthesis 2 of the second embodiment, in the present case a self-expanding oesophagus stent, is also preferably provided with a tube or stent coating 13 and is generally placed in the sheath 1 in the compressed state. The separation device is the same for both embodiments and is formed by inner pull element 9, outer pull element 10 and separating element 11. The inner pull element runs from the proximal end region of the catheter to the distal end region of the catheter next to the guide element 12 in the same lumen, then emerges from this innermost region through openings on the sheath wall and runs in the receiving region of the stent next to the sheath 1 directly within it.

In the region of the endoprosthesis 2, the sheath 1 is advantageously provided with a guide groove (not shown in more detail) for the inner pull element 9, which is aligned preferably axially, but possibly also in the shape of a helix or elongated spiral. This guide groove may on the one hand offer a secure and precise guide for the inner pull element 9, on the other hand the locally reduced wall thickness of the sheath due to the groove facilitates its separation in that it lowers the separating force required. Apart from the region of the groove, in particular in the region directly next to the groove, the sheath 1 has an adequately large wall thickness to provide the necessary counter force for separation and to design the sheath with adequate resistance to buckling.

Axially running grooves on the inner or outer side of the sheath on the remaining periphery, particularly on the opposite side of the cutting device enable the stent to open the cut-open sheath, since the dimensional stability is thus weakened in the peripheral direction. A similar effect can be achieved by using a specially profiled tube material with partially reduced wall thickness.

The sheath 1 is provided with a deflecting point 8 at its distal end, in which the separating device 3 emerges from the sheath from inside to out and is deflected from its distal path into an essentially proximal path. Shortly before the deflecting point 8, the inner pull element 9 merges into the separating element 11. After the deflecting point 8 the separating element 11 merges into the outer pull element 10. In the present case the separating device is designed to be integrated.

The outer pull element 10 is surrounded in the further path with shape precision by a protective tube 7. The outer pull element 10 runs together with the protective tube 7 through an opening 4 in the sheath wall into the interior of the catheter. In the preferred exemplary embodiment, outer pull element 10 and protective tube 7 penetrate the filler tubes 5, 6. The filler tubes shown have the task of holding the stent in position and reducing the buckling angle. The protective tube 7 stops shortly after this penetration. In their further path the inner 9 and outer 10 pull element use a common lumen and run directly next to the guide element 12.

In the present exemplary embodiment a wire made from stainless fine steel, preferably the metal alloy 1.4310, having a diameter of 0.15 mm has proved to be particularly advantageous as the separating element. Such dimensions are a particularly good compromise between a uniform separating effect and adequate ability to absorb tensile forces. However, it is also conceivable to use plastic materials, for example commercially available Angel cord at a diameter of 0.2 mm.

Although in the exemplary embodiment shown the separating device 3 is shown as an integrally designed wire, it has likewise been considered designing the wire to have several parts; a multi-part design is conceivable for example by producing inner and outer pull element 9, 10 from different materials and/or with dimensions different to the separating element 11. The present exemplary embodiment is based on a cutting effect due to shearing force; but it is likewise conceivable to utilise other mechanisms. For example the separating device and the pull elements could be made from metals having different electric conductivity values, and the separating element could be formed by the transition from one to the other metal. Exposing the separating device to voltage would produce heat in the separating element, by means of which the sheath can be separated.

The cutting wire may alternatively be flattened in the axial direction in the region of the separating element to increase the cutting effect. The cutting wire may on the other hand remain round in the regions of the pull elements to achieve an atraumatic effect.

The low-shearing-effect design of the outer pull element is realised in the present exemplary embodiment via tube 7. In the exemplary embodiment the tube is guided into the interior of the catheter together with the outer pull element 10. For this reason it is advantageous to produce the protective tube 7 from a material having a low friction coefficient, which can be sterilised simply at the same time - for example PTFE. It is likewise conceivable to design the outer pull element:, for example flat. For such a flat design a comparatively large surface would exist between the outer pull element and the surrounding vessel wall, so that when moving the outer pull element relative to the surrounding tissue the latter would not be damaged.

The function of the exemplary embodiment shown will be illustrated below using Figures 3 to 6.

In order to deploy the oesophagus stent 2 shown, a pulling action is made via the trigger mechanism 16 (Figure 1) simultaneously at the two pull elements 9, 10 in the direction of the arrows shown in Figures 2 and 3. A shearing or cutting effect is thus exerted on the sheath 1 at the deflecting point 8 via the separating element 11. On further pulling at the inner 9 and outer 10 pull element, the separating element 11 separates the sheath 1 in axial direction.

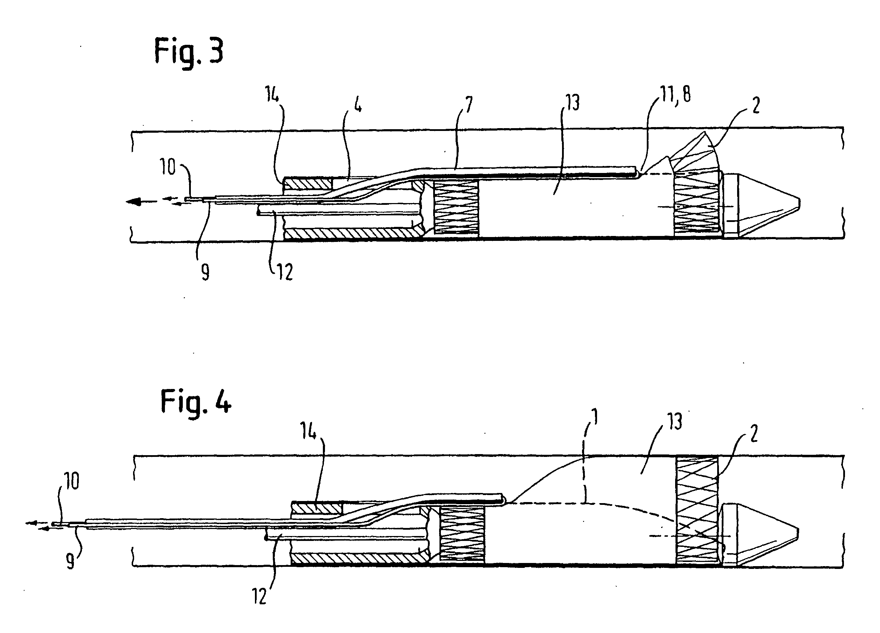

Figure 3 shows the initial disengagement of the stent 2. As can be seen, the stent 2 with stent coating 13 disengages laterally and is placed against the vessel wall (as indicated). In the further path of the triggering movement, the stent 2 is anchored in the tissue increasingly over an uninterrupted and considerable part of its periphery. The path of the separated part of the sheath 1 is shown in Figures 3 to 6 in broken lines.

Even in the state shown in Figure 3 it can be seen that the disengaging stent 2 presses the catheter gently against the opposite side of the vessel wall when it disengages from the sheath 1 and assists the action of the protective tube 7. The inner 9 and the outer 10 pull element are guided through the opening 4 in the sheath wall into the inner lumen of the catheter and run marginally proximally of the opening in the sheath wall next to the inner guide element 12.

From the further path shown in Figure 4 it becomes clear that the outer pull element 10 surrounded by the protective tube 7 together with the latter runs directly externally past the sheath 1 before it enters it. At no point in time is there the danger that one or both of the pull elements 9, 10 become dangerous to the vessel wall.

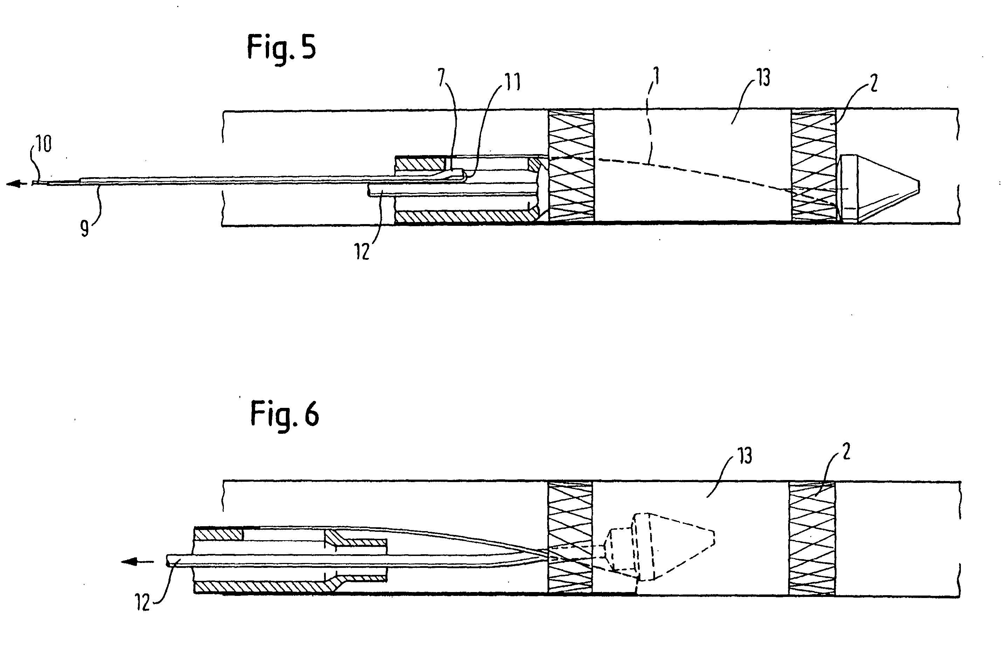

After complete triggering of the wire (see Figure 5), the sheath 1 is separated as far as the region of the opening 4 in the sheath wall and the stent is placed against the vessel wall over a considerable part of its periphery. The inner 9 and the outer pull element 10 together with the protective tube 7, as well as the separating element 11 are drawn into the catheter, so that no further components project externally beyond the sheath 1. The cut-open region of the sheath 1 lies on one side between the vessel wall and the expanded stent 2.

Due the complete running of the separating device 3 into the catheter, the user has noticeable trigger control since, after completed triggering, the separating device 3 glides freely through the catheter. On the other hand if the pull elements 9, 10 are allowed to run inwards through two separate openings through one or both filler tubes 5, 6 (not shown), after complete triggering the separating element remains suspended on the bar between these openings and cannot be triggered further. A noticeable end stop is thus realised for positive control.

As shown in Figure 6, the sheath 1 slides out between the stent 2 and the vessel wall when removing the catheter by pulling on the handle piece 15 (Figure 1) in the direction. of the arrow, while the stent 2 retains its position.

As investigations have shown, surprisingly, the position of the stent during placement does not change even for the basic embodiment of the present invention. Currently, this is explained in that the stent is buried in the flexible vessel wall and thus displacement of the stent is prevented by positive sheathing. Hence high placement accuracy is even provided when the position of the endoprosthesis or optionally radio-opaque tracers is monitored only once at the start of the placement process.

Traditional processes for deploying the stent by withdrawing the sheath lead, particularly for coated stents, to considerable friction problems. High surface pressing is produced due to the additional material in the sheath, the required recovery forces for deploying the stent firstly encounter the limits of the ability of the material to be stressed, secondly the forces can hardly be applied manually. These problems multiply with increasing stent length.

These problems are eliminated by the present invention. The triggering force of the separating device is very low, up to about 1/10 of the traditional triggering force, and virtually independent of the stent length. The material stress is low and - as tests have shown - a very precise placement of the stent is possible with the simplest manipulation.

The directions, such as inner and outer, given in the above description serve only to designate the relative position of the individual components with respect to one another, and have no further limiting meaning. Furthermore, the expert will be able to see that details of the various embodiments can also be combined with one another, even if this is not outlined in detail.
- 1: Sheath
- 2: Endoprosthesis/oesophagus stent
- 3: Separating device
- 4: Opening in the sheath wall
- 5: Filler tube
- 6: Filler tube
- 7: Tube/protective tube
- 8: Deflecting point
- 9: Inner pull element
- 10: Outer pull element
- 11: Separating element/cutting wire
- 12: Inner guide element/inner catheter
- 13: Stent coating
- 14: Integrated filler
- 15: Handle piece
- 16: Trigger mechanism
- 20: Application instrument

## Claims

1. Catheter for introducing and placing an endoprosthesis in a human or animal body having a receiving region for the endoprosthesis (2), a sheath (1) surrounding the catheter externally and a device (3) for separating the sheath; the separating device (3) has an inner pull element (9), an outer pull element (10) and a separating element (11);
the inner pull element (9) runs within the sheath (1) in the region of the catheter which can be introduced;
the outer pull element (10) runs outside the sheath (1) only in the receiving region for the endoprosthesis (2);
when actuating the separating device (3), the inner pull element (9), the outer pull element (10) and the separating element (11) can be moved together relative to the sheath (1) to separate the sheath,
**characterized in that**
the outer pull element (10) is covered to have a low shearing effect in its region running outside the sheath, such that mechanical damage to the tissue surrounding the catheter is prevented.

2. Catheter according to claim 1, **characterized in that** the separating element (11) has a length which essentially corresponds to the wall thickness of the sheath (1).

3. Catheter according to claims 1 or 2, **characterized in that** the outer and inner pull element (9, 10) are integrally formed and a portion thereof is formed as the separating element (3).

4. Catheter according to at least one of the preceding claims, **characterized in that** the sheath (1) comprises a guiding groove for the inner pull element (9).

5. Catheter according to at least one of the preceding claims, **characterized in that** only one single separating device (3) is provided.

6. Catheter according to at least one of the preceding claims, **characterized in that** the separating element is a cutting wire (11).

7. Catheter according to at least one of the preceding claims, **characterized in that** the separating device (3) runs in a loop from a proximal end region to a distal end region of the catheter, around a deflecting point (8) and back to the proximal end region of the catheter.

8. Catheter according to claim 7, **characterized in that** the separating element (11) is arranged at the deflecting point (8).

9. Catheter according to at least one of the preceding claims, **characterized in that** the separating element (11) is directly distally next to a distal end of the sheath (1).

10. Catheter according to claim 9, **characterized in that** the separating element (11) is fixed tangentially in the distal end region of the sheath (1) via positive sheathing.

11. Catheter according to at least one of the preceding claims, **characterized in that** the covered region of the pull element (10) running outside the sheath and the separating element (11) are formed from a single length of wire.

12. Catheter according to claim 11, **characterized in that** the region of the pull element (10) running outside the sheath is covered by a tube (7).

13. Catheter according to at least one of the preceding claims, **characterized in that** the catheter comprises an inner guide element (12) and the separating device (3) runs proximally of the receiving region for the endoprosthesis (2) next to the inner guide element (12).

14. Catheter according to at least one of the preceding claims, **characterized in that** a control device displaying the end of separation is provided.

15. Application instrument having a catheter according to at least one of the preceding claims and an endoprosthesis (2).

16. Application instrument according to claim 15, wherein the endoprosthesis (2) is an oesophagus stent.

17. Application instrument according to claim 16, wherein the oesophagus stent is self-expanding.

## Patentansprüche

1. Katheter zum Einführen und Platzieren einer Endoprothese in einen menschlichen oder tierischen Körper, der einen Aufnahmebereich für die Endoprothese (2), einen den Katheter von außen umgebenden Mantel (1) und eine Vorrichtung (3) zum Trennen des Mantels besitzt, bei dem die Trennvorrichtung (3) ein inneres Zugelement (9), ein äußeres Zugelement (10) und ein Trennelement (11) besitzt; und
das innere Zugelement (9) innerhalb des Mantels (1) in dem Bereich des Katheters, der eingeführt werden kann, verläuft;
das äußere Zugelement (10) außerhalb des Mantels (1) lediglich in dem Aufnahmebereich für die Endoprothese (2) verläuft;
beim Betätigen der Trennvorrichtung (3) das innere Zugelement (9), das äußere Zugelement (10) und das Trennelement (11) zusammen relativ zu dem Mantel (1) bewegt werden können, um den Mantel zu trennen;
**dadurch gekennzeichnet, dass**
das äußere Zugelement (10) derart überzogen ist, dass sein außerhalb des Mantels verlaufender Bereich eine geringe Scherwirkung zeigt, so dass eine mechanische Beschädigung des den Katheter umgebenden Gewebes vermieden wird.

2. Katheter nach Anspruch 1, **dadurch gekennzeichnet, dass** das Trennelement (11) eine Länge besitzt, die im Wesentlichen der Wandstärke des Mantels (1) entspricht.

3. Katheter nach Anspruch 1 oder 2, **dadurch gekennzeichnet, dass** das äußere und das innere Zugelement (9, 10) integral ausgebildet sind und ein Abschnitt derselben als das Trennelement (3) ausgebildet ist.

4. Katheter nach mindestens einem der vorhergehenden Ansprüche, **dadurch gekennzeichnet, dass** der Mantel (1) eine Führungsnut für das innere Zugelement (9) aufweist.

5. Katheter nach mindestens einem der vorhergehenden Ansprüche, **dadurch gekennzeichnet, dass** nur eine einzelne Trennvorrichtung (3) vorgesehen ist.

6. Katheter nach mindestens einem der vorhergehenden Ansprüche, **dadurch gekennzeichnet, dass** das Trennelement ein Schneiddraht (11) ist.

7. Katheter nach mindestens einem der vorhergehenden Ansprüche, **dadurch gekennzeichnet, dass** die Trennvorrichtung (3) schlaufenförmig von einem proximalen Endbereich zu einem distalen Endbereich des Katheters, um einen Umlenkpunkt (8) herum und zurück zu dem proximalen Endbereich des Katheters verläuft.

8. Katheter nach Anspruch 7, **dadurch gekennzeichnet, dass** das Trennelement (11) an dem Umlenkpunkt (8) angeordnet ist.

9. Katheter nach mindestens einem der vorhergehenden Ansprüche, **dadurch gekennzeichnet, dass** das Trennelement (11) unmittelbar distal eines distalen Endes des Mantels (1) gelegen ist.

10. Katheter nach Anspruch 9, **dadurch gekennzeichnet, dass** das Trennelement (11) in tangentialer Richtung in dem distalen Endbereich des Mantels (1) durch eine Ummantelung fixiert ist.

11. Katheter nach mindestens einem der vorhergehenden Ansprüche, **dadurch gekennzeichnet, dass** der überzogene Bereich des Zugelements (10), der außerhalb des Mantels verläuft, und das Trennelement (11) aus einem einzelnen Drahtlänge gebildet sind.

12. Katheter nach Anspruch 11, **dadurch gekennzeichnet, dass** der Bereich des Zugelements (10), der außerhalb des Mantels verläuft, durch einen Schlauch (7) überzogen ist.

13. Katheter nach mindestens einem der vorhergehenden Ansprüche, **dadurch gekennzeichnet, dass** der Katheter ein inneres Führungselement (12) aufweist und die Trennvorrichtung (3) in proximaler Richtung von dem Aufnahmebereich für die Endoprothese (2) neben dem inneren Führungselement (12) verläuft.

14. Katheter nach mindestens einem der vorhergehenden Ansprüche, **dadurch gekennzeichnet, dass** eine Kontrollvorrichtung vorgesehen ist, die das Ende des Trennvorgangs anzeigt.

15. Applikationsinstrument mit einem Katheter nach mindestens einem der vorhergehenden Ansprüche und einer Endoprothese (2).

16. Applikationsinstrument nach Anspruch 15, bei der die Endoprothese (2) ein Ösophagus-Stent ist.

17. Applikationsinstrument nach Anspruch 16, bei der der Ösophagus-Stent selbst-expandierend ist.

## Revendications

1. Cathéter pour l'introduction et la mise en place d'une endoprothèse dans un corps humain ou animal comprenant une région de réception pour l'endoprothèse (2), une gaine (1) entourant le cathéter de manière externe et un dispositif (3) pour séparer la gaine ; le dispositif de séparation (3) a un élément de traction interne (9), un élément de traction externe (10) et un élément de séparation (11) ;
l'élément de traction interne (9) s'étend dans la gaine (1) dans la région du cathéter qui peut être introduit ;
l'élément de traction externe (10) s'étend à l'extérieur de la gaine (1) uniquement dans la région de réception pour l'endoprothèse (2) ;
lors de l'actionnement du dispositif de séparation (3), l'élément de traction interne (9), l'élément de traction externe (10) et l'élément de séparation (11) peuvent être déplacés ensemble par rapport à la gaine (1) pour séparer la gaine,
**caractérisé en ce que**
l'élément de traction externe (10) est recouvert pour avoir un faible effet de cisaillement dans sa région s'étendant à l'extérieur de la gaine, de sorte que l'on empêche l'endommagement mécanique du tissu entourant le cathéter.

2. Cathéter selon la revendication 1, **caractérisé en ce que** l'élément de séparation (11) a une longueur qui correspond essentiellement à l'épaisseur de paroi de la gaine (1).

3. Cathéter selon les revendications 1 ou 2, **caractérisé en ce que** les éléments de traction externe et interne (9, 10) sont formés de manière solidaire et une partie de ceux-ci est formée en tant qu'élément de séparation (3).

4. Cathéter selon au moins l'une des revendications précédentes, **caractérisé en ce que** la gaine (1) comprend une rainure de guidage pour l'élément de traction interne (9).

5. Cathéter selon au moins l'une des revendications précédentes, **caractérisé en ce que** l'on ne prévoit qu'un seul dispositif de séparation (3).

6. Cathéter selon au moins l'une des revendications précédentes, **caractérisé en ce que** l'élément de séparation est un fil de coupe (11).

7. Cathéter selon au moins l'une des revendications précédentes, **caractérisé en ce que** le dispositif de séparation (3) s'étend dans une boucle d'une région d'extrémité proximale à une région d'extrémité distale du cathéter, autour d'un point de déviation (8) et à nouveau vers la région d'extrémité proximale du cathéter.

8. Cathéter selon la revendication 7, **caractérisé en ce que** l'élément de séparation (11) est agencé au niveau du point de déviation (8).

9. Cathéter selon au moins l'une des revendications précédentes, **caractérisé en ce que** l'élément de séparation (11) est de manière distale directement successif à une extrémité distale de la gaine (1).

10. Cathéter selon la revendication 9, **caractérisé en ce que** l'élément de séparation (11) est fixé de manière tangentielle dans la région d'extrémité distale de la gaine (1) via un gainage positif.

11. Cathéter selon au moins l'une des revendications précédentes, **caractérisé en ce que** la région recouverte de l'élément de traction (10) s'étendant à l'extérieur de la gaine et l'élément de séparation (11) sont formés à partir d'une longueur unique de fil.

12. Cathéter selon la revendication 11, **caractérisé en ce que** la région de l'élément de traction (10) s'étendant à l'extérieur de la gaine est recouverte par un tube (7).

13. Cathéter selon au moins l'une des revendications précédentes, **caractérisé en ce que** le cathéter comprend un élément de guide interne (12) et le dispositif de séparation (3) s'étend de manière proximale par rapport à la région de réception pour l'endoprothèse (2) suite à l'élément de guide interne (12).

14. Cathéter selon au moins l'une des revendications précédentes, **caractérisé en ce que** l'on prévoit un dispositif de commande affichant l'extrémité de séparation.

15. Instrument d'application doté d'un cathéter selon au moins l'une des revendications précédentes et une endoprothèse (2).

16. Instrument d'application selon la revendication 15, dans lequel l'endoprothèse (2) est un stent oesophagien.

17. Instrument d'application selon la revendication 16, dans lequel le stent oesophagien est auto-expansible.
